(19) Europäisches Patentamt
European Patent Office
Office européen des brevets

(11) EP 1 408 116 A1

(12) **EUROPEAN PATENT APPLICATION**
published in accordance with Art. 158(3) EPC

(43) Date of publication:
**14.04.2004 Bulletin 2004/16**

(21) Application number: **02705414.7**

(22) Date of filing: **20.03.2002**

(51) Int Cl.[7]: **C12P 19/64**, C07H 11/00, C07H 15/04, C07H 15/203, C08B 37/00

(86) International application number:
**PCT/JP2002/002716**

(87) International publication number:
**WO 2002/103025 (27.12.2002 Gazette 2002/52)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **14.06.2001 JP 2001180163**

(71) Applicant: **National Institute of Advanced Industrial Science and Technology**
**Tokyo 100-0013 (JP)**

(72) Inventors:
- **UZAWA, Hirotaka,**
**c/o Tsukuba Center National Inst.**
**1-1-1, Higashi, Tsukuba-shi, Ibaraki (JP)**
- **MINOURA, Norihiko,**
**c/o Tsukuba Center Nat. Inst.**
**1-1-1, Higashi, Tsukuba-shi, Ibarak (JP)**
- **ZENG, Xiaoxiong,**
**c/o Tsukuba Center National Inst.**
**1-1-1, Higashi, Tsukuba-shi, Ibaraki (JP)**

(74) Representative:
**Luderschmidt, Schüler & Partner GbR**
**Patentanwälte,**
**Postfach 3929**
**65029 Wiesbaden (DE)**

# (54) NOVEL SULFATED SACCHARIDE AND PROCESS FOR PRODUCING THE SAME

(57) A sulfated saccharide represented by the following general formula (I):

$$[A] - (6SO_3 - GlcNAc) - [C] - R \qquad (I)$$

wherein ($6SO_3$ - GlcNAc) represents a 6-sulfated N-acetylglucosamine residue, [A] represents a hydroxyl group or a saccharide residue, [C] represents a saccharide residue and R represents a hydroxyl group or a substituted hydroxyl group is disclosed. The sulfated saccharide effectively bonds to selectin protein which is present in leukocytes and hence has anti-inflammatory activity. The sulfated saccharide is expected to be widely used in the medicinal field, etc.

EP 1 408 116 A1

## Description

TECHNICAL FIELD:

The present invention relates to novel sulfated saccharides and a process for preparing the same.

BACKGROUND ART

As saccharides and glycolipids having a sulfate group in their molecules, sulfated sialyl-Lewis X, sulfatides, etc. are known. They are also known to specifically bind with selectin proteins which are present in human and animal leukocytes. Thus, the sulfated saccharides which show selectin blockers are expected to be developed and utilized as medicines such as anti-inflammatory medicines and cancer metastasis-preventing medicines.

Various sulfated saccharides and methods for the preparation thereof have been hitherto developed. Such methods include as follows:

(1) method of recovering from natural products by separation methods such as extraction and chromatography;
(2) method using enzymes;
(3) method by chemical synthesis; and
(4) method combining the above methods (2) and (3).

With the method (1) above, the desired product is generally obtained in only a trace amount. While the method (3) above can give the desired product in a large amount, much labors are required because the method includes many reaction steps. Concerning the method (2) above, the are relatively a large number of reports pertaining to the synthesis of sulfate group-free saccharides. Only a few synthesis examples are known with regard to sulfate group-containing saccharides.

Incidentally, known methods of synthesizing sulfated saccharides using enzymes may be classified into two groups depending upon the kind of enzymes used.

(a) Synthesis using sulfotransferase

$$\mathrm{GlcNAc\beta 1{\rightarrow}4GlcNAc} \rightarrow \mathrm{GlcNAc\beta 1{\rightarrow}4(6SO_3)GlcNAc}$$

$$\mathrm{GlcNAc\beta 1{\rightarrow}4GlcNAc\beta 1{\rightarrow}4GlcNAc\beta 1{\rightarrow}4GlcNAc} \rightarrow$$

$$\mathrm{GlcNAc\beta 1{\rightarrow}4GlcNAc\beta 1{\rightarrow}4GlcNAc\beta 1{\rightarrow}4(6SO_3)GlcNAc}$$

The above synthesis method is disclosed in C.-H. Wong, J. Org. Chem., 65, 5565-5574 (2000); J. Am. Chem. Soc., 117, 8031 (1955).

(b) Synthesis using galactotransferase

$$\mathrm{6SO_3\text{-}GlcNAc\beta 1{\rightarrow}3Gal\beta 1{\rightarrow}4Glc\beta{\rightarrow}OMPM} \rightarrow$$

$$\mathrm{Gal\beta 1{\rightarrow}44\ (6SO_3)GlcNAc\beta 1{\rightarrow}3Gal\beta 1{\rightarrow}4Glc\beta{\rightarrow}OMPM}$$

The above synthesis method is disclosed in Lubineau, Carbohydrate Res., 305, 510 (1998).

The reactions using the above enzymes are indicated as being useful as reactions per se but encounter great difficulties in actually carrying out the reactions.

The method (a) is characterized in the use of a transferase enzyme (sulfotransferase) capable of transferring a sulfate group. The transferase enzyme is, however, expensive so that the sulfated saccharide product produced by using the expensive enzyme is unavoidably expensive too. Thus, such a method cannot be utilized for actual production on an industrial scale because of the production costs. Further, the position to which a sulfate group is transferred is

limited to a 6 position on the reducing terminal side, i.e. it is difficult to synthesize sulfated saccharides in which the sulfate group is introduced to a non-reducing terminal. Therefore, the method (a) cannot be said as being a practical method.

In the method (b), a transferase enzyme (galactotransferase) capable of transferring a sulfate group is used. The enzyme per se is currently used in various reactions and is in an already developed state. This enzyme, too, is expensive. Further, the substrate utilizable for this method is considerably limited. Therefore, the method (b) cannot be said to be a practical method.

The objects of the present invention are as follows:

(1) to provide a novel sulfated saccharide having a structure in which a sulfated saccharide chain is introduced into a non-reducing terminal of a saccharide, and to provide a method of producing the sulfated saccharide; and
(2) to provide a novel sulfated saccharide having a structure in which a sulfated saccharide chain is introduced into a reducing terminal of a saccharide, and to provide a method of producing the sulfated saccharide.

DISCLOSURE OF THE INVENTION

The present inventors have found that the reaction of a sulfated saccharide as a donor with "another saccharide" as an acceptor in the presence of N-acetylhexosaminidase or N-acetylglucosaminidase as an enzyme can introduce a sulfated saccharide chain to a non-reducing terminal of the "another saccharide". Similarly, the reaction of a sulfated saccharide as an acceptor with "another saccharide" as a donor has been found to introduce a sulfated saccharide chain to the reducing terminal of the "another saccharide".

The N-acetylhexosaminidase (or N-acetylglucosaminidase) used in each of the above reactions is substantially the same. By using this enzyme, the above reactions can be accelerated. Since this enzyme is easily feasible and has no economical problems, the above reactions can be carried out on an industrial scale in an advantageous manner.

In accordance with the present invention, there are provided the following novel sulfated saccharides and process of producing same.

(1) A sulfated saccharide represented by the following general formula (I):

$$[A] - (6SO_3 - GlcNAc) - [C] - R \qquad (I)$$

wherein ($6SO_3$ - GlcNAc) represents a 6-sulfated N-acetylglucosamine residue, [A] represents a hydroxyl group or a saccharide residue, [C] represents a saccharide residue and R represents a hydroxyl group or a substituted hydroxyl group.

(2) A sulfated saccharide as recited in the above item (1), wherein the saccharide residue represented by [A] of the general formula (I) is a saccharide residue derived from a saccharide selected from glucose, galactose, mannose, N-acetylglucosamine, N-acetylgalactosamine, N-acetylmannosamine and oligosaccharides thereof.

(3) A sulfated saccharide as recited in the above item (1) or (2), wherein the substituted hydroxyl group represented by [C] of the general formula (I) is p-nitrophenoxy group.

(4) A sulfated saccharide as recited in any one of the above items (1) to (3), wherein the saccharide residue represented by [A] contains a sulfate group or a phosphate group.

(5) A sulfated saccharide as recited in any one of the above items (1) to (4), wherein the saccharide residue represented by [C] of the general formula (I) is a saccharide residue derived from a saccharide selected from glucose, galactose, mannose, N-acetylglucosamine, N-acetylgalactosamine, N-acetylmannosamine and oligosaccharides thereof.

(6) A sulfated saccharide as recited in the above item (5), wherein the saccharide residue represented by [C] contains a sulfate group or a phosphate group.

(7) A sulfated saccharide represented by the following general formula (II):

$$HO - (GlcNAc) - (6SO_3 - GlcNAc) - [D] \qquad (II)$$

wherein ($6SO_3$ - GlcNAc) represents a 6-sulfated N-acetylglucosamine residue, (GlcNAc) represents an N-acetylglucosamine residue, and [D] represents a hydroxyl group, a substituted hydroxyl group or a saccharide residue.

(8) A sulfated saccharide as recited in the above item (7), wherein the saccharide residue represented by (D] of the general formula (II) is a saccharide residue derived from a saccharide selected from glucose, galactose, mannose, N-acetylglucosamine, N-acetylgalactosamine, N-acetylmannosamine and oligosaccharides thereof.
(9) A sulfated saccharide as recited in the above item (7) or (8), wherein the substituted hydroxyl group represented by [D] of the general formula (II) is p-nitrophenoxy group.
(10) A sulfated saccharide as recited in any one of the above items (7) to (9), wherein the saccharide residue represented by [D] contains a sulfate group or a phosphate group.
(11) A process of preparing a sulfated saccharide represented by the following general formula (I):

$$[A] - (6SO_3 - GlcNAc) - [C] - R \qquad (I)$$

wherein ($6SO_3$ - GlcNAc) represents a 6-sulfated N-acetylglucosamine residue, [A] represents a hydroxyl group or a saccharide residue, [C] represents a saccharide residue and R represents a hydroxyl group or a substituted hydroxyl group, characterized in that a sulfated saccharide represented by the following general formula (Ia):

$$[A] - (6SO_3 - GlcNAc) - [B] \qquad (Ia)$$

wherein ($6SO_3$ - GlcNAc) and [A] have the same meaning as above and [B] represents a hydroxyl group or a substituted hydroxyl group,
is reacted with a saccharide of the following general formula (Ib):

$$HO - [C] - R \qquad (Ib)$$

wherein [C] represents a saccharide residue and R represents a hydroxyl group, a substituted hydroxyl group or a saccharide residue, in the presence of N-acetylhexosaminidase or N-acetylglucosaminidase.
(12) A process of preparing a sulfated saccharide represented by the following general formula (II):

$$HO - (GlcNAc) - (6SO_3 - GlcNAc) - [D] \qquad (II)$$

wherein ($6SO_3$ - GlcNAc) represents a 6-sulfated N-acetylglucosamine residue, (GlcNAc) represents an N-acetylglucosamine residue, and [D] represents a hydroxyl group, a substituted hydroxyl group or a saccharide residue, characterized in that a sulfated saccharide represented by the following general formula (IIa):

$$HO - (6SO_3 - GlcNAc) - [D] \qquad (IIa)$$

wherein ($6SO_3$-GlcNAc) and [D] have the same meaning as above,
is reacted with a saccharide of the following general formula (IIb) :

$$HO - [GlcNAc] - [Z] \qquad (IIb)$$

wherein [GlcNAc] represents an N-acetylglucosamine residue and [Z] represents a hydroxyl group, a substituted hydroxyl group or a saccharide residue,
in the presence of N-acetylhexosaminidase or N-acetylglucosaminidase.

In accordance with the first aspect of the present invention, there is provided a process of preparing a sulfated saccharide represented by the following general formula (I):

$$[A] - (6SO_3 - GlcNAc) - [C] - R \qquad (I)$$

wherein ($6SO_3$-GlcNAc) represents a 6-sulfated N-acetylglucosamine residue.

The above symbol [A] represents a hydroxyl group or a saccharide residue. The saccharide residues include residues derived from saccharides selected from monosaccharides, oligosaccharides and polysaccharides. The monosaccharide may be a conventionally known monosaccharide, such as glucose, galactose, mannose, N-acetylglucosamine, N-acetylgalactosamine and N-acetylmannosamine. The oligosaccharides may include those of the above monosaccharides, chondroitin sulfate, dermatan sulfate and heparin. The polysaccharides may include those obtained by highly polymerizing the above monosaccharides or oligosaccharides. These saccharides may contain sulfate groups or phosphate groups.

The above symbol [C] represents a saccharide residue. The saccharide residues include residues derived from saccharides selected from monosaccharides, oligosaccharides and polysaccharides. The monosaccharide may be a conventionally known monosaccharide, such as glucose, galactose, mannose, N-acetylglucosamine, N-acetylgalactosamine and N-acetylmannosamine. The oligosaccharides may include those of the above monosaccharides, chondroitin sulfate, dermatan sulfate and heparin. The polysaccharides may include those obtained by highly polymerizing the above monosaccharides or oligosaccharides. These saccharides may contain sulfate groups or phosphate groups.

The symbol R represents a hydroxyl group or a substituted hydroxyl group. The term "substituted hydroxyl group" as used herein is intended to refer to a hydroxyl group having the hydrogen substituted with a substituent and is represented by -OR' in which R' may be an aliphatic group or aromatic group. The aliphatic group may contain an unsaturated bond and may contain 1 to 4 carbon atoms. Examples of the aliphatic group include methyl, ethyl, propyl and butyl. The aromatic group may be phenyl or substituted phenyl. As the substituted phenyl, there may be mentioned phenyl having a nitro group, an alkoxy group or the like as the substituent. Preferred substituted phenyl is p-nitrophenyl or o-nitrophenyl.

In accordance with the second aspect of the present invention, there is provided a process of preparing a sulfated saccharide represented by the following general formula (II):

$$HO - (GlcNAc) - (6SO_3 - GlcNAc) - [D] \qquad (II)$$

wherein ($6SO_3$-GlcNAc) has the same meaning as above and (GlcNAc) represents a residue derived from N-acetylglucosamine.

The above symbol [D] represents a hydroxyl group, a substituted hydroxyl group or a saccharide residue. The saccharide residues include residues derived from saccharides selected from monosaccharides, oligosaccharides and polysaccharides. The monosaccharide may be a conventionally known monosaccharide, such as glucose, galactose, mannose, N-acetylglucosamine, N-acetylgalactosamine and N-acetylmannosamine. The oligosaccharides may include those of the above monosaccharides, chondroitin sulfate, dermatan sulfate and heparin. The polysaccharides may include those obtained by highly polymerizing the above monosaccharides or oligosaccharides. These saccharides may contain sulfate groups or phosphate groups. The term "substituted hydroxyl group" as used herein is intended to refer to a hydroxyl group having the hydrogen substituted with a substituent. Specific examples of the substituted hydroxyl group include those described above.

The sulfated saccharide represented by the general formula (I) is produced by reacting a sulfated saccharide represented by the following general formula (Ia):

$$[A] - (6SO_3 - GlcNAc) - [B] \qquad (Ia)$$

wherein ($6SO_3$-GlcNAc) and [A] have the same meaning as above and [B] represents a hydroxyl group or a substituted hydroxyl group,
with a saccharide of the following general formula (Ib):

$$HO - [C] - R \qquad (Ib)$$

wherein [C] represents a saccharide residue and R represents a hydroxyl group or a substituted hydroxyl group, in the presence of N-acetylhexosaminidase or N-acetylglucosaminidase.

The above reaction is shown by the following formula:

$$[A] - (6SO_3 - GlcNAc) - [B] + HO - [C] - R$$

$$\rightarrow [A] - (6SO_3 - GlcNAc) - [C] - R + B \qquad (a)$$

In the above general formula (Ia), the bonding style between [B] and ($6SO_3$-GlcNAc) may be either α-linkage or β-linkage, in which [B] is linked to the 1 position of ($6SO_3$-GlcNAc). When [B] is a residue derived from a saccharide selected from monosaccharides and oligosaccharides, [B] is linked at the 2 position, 3 position, 4 position or 6 position thereof to the 1 position of ($6SO_3$-GlcNAc). When [B] is a residue derived from N-acetylglucosamine or N-acetylmannosamine, [B] is linked at the 3 position, 4 position or 6 position thereof (other than the 1 position) to the 1 position of ($6SO_3$-GlcNAc).

The sulfated saccharide represented by the above general formula (IIa) is a known compound and is commercially available as a natural or synthetic product. Specific examples of the sulfated saccharide represented by the above general formula (IIa) include:

(1) HO - ($6SO_3$ - GlcNAc) - $OCH_3$
(2) HO - ($6SO_3$ - GlcNAc) - $OC_2H_5$
(3) HO - ($6SO_3$ - GlcNAc) - $OC_6H_4NO_2$
(the position of $NO_2$ is preferably ortho- or para-position)
(4) HO - [Gal] - ($6SO_3$ - GlcNAc) - $OCH_3$
(5) HO - [Gal] - ($6SO_3$ - GlcNAc) - $OC_2H_5$
(6) HO - [Gal] - ($6SO_3$ - GlcNAc) - $OC_6H_4NO_2$
(the position of $NO_2$ is preferably ortho- or para-position).

In the above formulas, [Gal] designates a galactose residue. The galactose residue [Gal] may be replaced by a glucose residue, a mannose residue, an N-acetylglucosamine reside, an N-acetylgalactosamine residue, an N-acetylmannosamine residue or a residue of an oligosaccharide thereof.

The structural formula of [A] - ($6SO_3$ - GlcNAc) - [B] is as shown below.

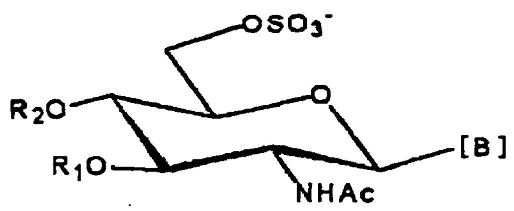

(Ia)

In the above formula, the following combinations of $R_1$ and $R_2$ are possible:

(1) $R_1$ = [A] and $R_2$ = H
(2) $R_1$ = H and $R_2$ = [A]
(3) $R_1$ = H and $R_2$ = H (when [A] is OH)

The structural formula of the saccharide residue represented by [A] is as shown below.

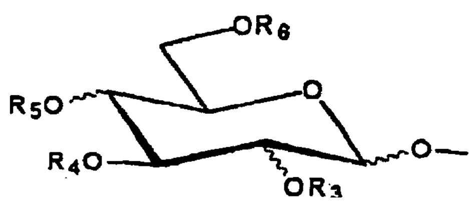

[A]

In the above formula, $R_3$ to $R_6$ are each hydrogen, or one of $R_3$ to $R_6$ is a sulfate group, a phosphate group or a saccharide residue with the remainder groups being each hydrogen. The saccharide residues include residues derived from monosaccharides such as glucose and galactose, and residues derived from oligosaccharides of the monosaccharides.

The structural formula of the saccharide residue represented by [B] is as shown below.

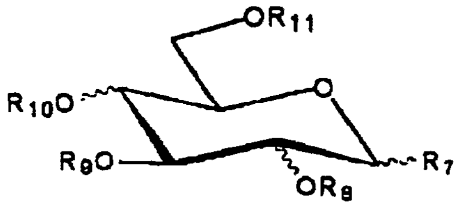

[B]

In the above formula, $R_7$ represents an alkyl group, a phenyl group, a substituted phenyl group, hydrogen or a saccharide residue. One of $R_8$ to $R_{11}$ is a direct bond (-) and is linked to the 1 position (the position to which [B] of the formula Ia is linked) of ($6SO_3$-GlcNAc), with the remainder groups being hydrogen atoms. One of the hydrogen atoms may be replaced by a sulfate group or a phosphate group.

In the above formulas [A] and [B], $R_3O$-, $R_5O$-, $R_7$-, $R_8O$- and $R_{10}O$- may be either axial or equatorial conformation.

In the above general formulas (I) and (Ia), the linkage between [A] and ($6SO_3$-GlcNAc) may be either α-linkage or β-linkage, in which [A] is linked at its 1 position to the 3 position or 4 position of ($6SO_3$-GlcNAc).

In the saccharide residue represented by [A], the 6 position, 2 position, 3 position or 4 position may be sulfated or phosphated. The sulfated or phosphated saccharides may include, for example, galactose sulfated at its 3 position and glucose phosphated at its 6 position.

In the above [C]-R, [C] represents a saccharide residue. The saccharide residues include residues derived from saccharides selected from monosaccharides, oligosaccharides and polysaccharides. The monosaccharide may be a conventionally known monosaccharide, such as glucose, galactose, mannose, N-acetylglucosamine, N-acetylgalactosamine and N-acetylmannosamine. The oligosaccharides may include those of the above monosaccharides, chondroitin sulfate, dermatan sulfate and heparin. The polysaccharides may include those obtained by highly polymerizing the above monosaccharides or oligosaccharides.

The symbol R represents a hydroxyl group or a substituted hydroxyl group. Examples of the substituted hydroxyl group include those described previously.

In the saccharide residue represented by [C], the hydroxyl group at the 1 position may be a substituted hydroxyl group. Examples of the substituted hydroxyl group include those described previously.

The structural formula of an example of the saccharide residue represented by [C] is as shown below.

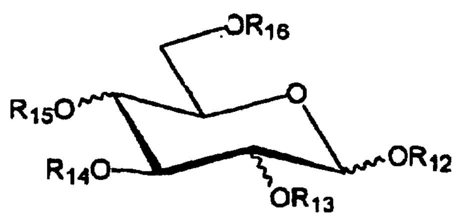

[C]

In the above formula, $R_{12}$ represents hydrogen, a substituent or a saccharide residue. One of $R_{13}$ to $R_{16}$ is a direct bond (-) with the remainder groups being hydrogen atoms. One of the hydrogen atoms may be replaced by a saccharide residue, a sulfate group or a phosphate group. $OR_{13}$ may be NHAc. Examples of the saccharide giving the saccharide reside [C] include methyl α-glucopyranoside, lactose (galactopyranoxylβ1→4glucopyranoside) and β-D-N-acetylglucosamine.

In the present invention, the sulfated saccharide of the above general formula (Ia) is reacted with the saccharide of the above general formula (Ia). The reaction in this case is a reaction for transferring the sulfate group ($SO_3$)-containing saccharide.

The amount of the raw materials is generally 1:1 in molar ratio, though one of them can be used in an excess amount. The reaction is performed in a liquid phase at a temperature in the range of 0 to 80°C, preferably between room temperature and 50°C. N-acetylhexosaminidase (or N-acetylglucosaminidase) is used as an enzyme. The enzyme may be derived from mold, bovine internal organs, bacteria, bean, yeast, recombinant DNA, etc.

The enzyme N-acetylhexosaminidase (E.C. 3.2.1.52) or or N-acetylglucosaminidase (E.C. 3.2.1.52) (these enzymes are substantially the same but commercial products thereof are in the marketplace under different names) may be used in a state in which it is supported on a support. When the enzyme is supported on a support, the process can be carried out at low costs. As the support, there may be mentioned an alginic acid resin and liposome.

The reaction is carried out in the presence of a solvent which is generally an aqueous medium. After completion

of the reaction, the reaction product is isolated with a yield of 1 to 80 % based on the raw materials. After completion of the reaction, the reaction product is separated and purified by means of reverse chromatography, molecular sieves or ion exchange.

In the above reaction, a customarily employed buffer solution such as phosphate butter, TRIS buffer or HEPES buffer. In the buffer solution, a hydrophilic solvent such as acetonitrile or methanol may be added in an amount of 0 to 90 %, preferably 10 to 50 %. Although a rare case, a solvent such as toluene or diethyl ether is used. In this case, the reaction is in a two-phase system and is performed at a temperature in the range of 0 to 80°C, preferably between about room temperature and 50°C.

In the product [A]-($6SO_3$-GlcNAc)-[C]-R obtained in the above reaction, the bonding style between ($6SO_3$-GlcNAc) and [C] may be any one of β1→2, β1→3, β1→4, β1→6, α1→2, α1→3, α1→4 and α1→6, or a mixture thereof. When [C] is N-acetylglucosamine, N-acetylgalactosamine or N-acetylmannosamine, β1→2 and α1→2 styles are excluded. When [C] contains a sulfate group or a phosphate group, the linkage to the position at which such an anionic group exists is excluded.

The linkage between [A] and ($6SO_3$ - GlcNAc) may be either α-linkage or β-linkage, in which [A] is linked at its 1 position to the 3 position or 4 position of ($6SO_3$-GlcNAc).

Specific examples of the sulfated saccharide represented by the above general formula (I) include:

(1) HO-β-D-(6-sulfo)- GlcNAc-(1→4)-α-Glc-$OCH_3$
(2) HO-(6-sulfo)-GlcNAcβ-(1→3 or 6)-Galβ-1→4-Glc
(3) HO-(6-sulfo)-GlcNAcβ-(1→3 or 4)-GlcNAc

The compound of the general formula (I) is characterized by a structure which is obtained by reaction of a sulfated saccharide as a saccharide chain donor with "another saccharide" as a saccharide chain acceptor and in which the sulfated saccharide chain is introduced into the non-reducing terminal for extending the chain. The byproduct corresponding to the desired product is a saccharide [B]. The compound of the general formula (I) effectively bonds to a selectin protein which is present in leukocytes and hbnce has anti-inflammatory activity. The compound is expected to be widely used in the medicinal field, etc.

The sulfated saccharide represented by the above general formula (II) may be produced by reacting a sulfated saccharide represented by the following general formula (IIa):

$$\text{HO - (6SO}_3\text{ - GlcNAc) - [D]} \qquad \text{(IIa)}$$

with a saccharide represented by the following general formula (IIb):

$$\text{HO - [GlcNAc] - [Z]} \qquad \text{(IIb)}$$

in the presence of the above-mentioned enzyme.

The above reaction is shown by the following formula:

$$\text{HO - (6SO}_3\text{ - GlcNAc) - [D] + HO - [GlcNAc] - [Z]}$$

$$\rightarrow \text{HO - [GlcNAc] - (6SO}_3\text{ - GlcNAc) - [D] + [Z]} \qquad \text{(b)}$$

The above symbol [D] represents a hydroxyl group, a substituted hydrpxyl group or a saccharide residue. The saccharide residues include residues derived from saccharides selected from monosaccharides, oligosaccharides and polysaccharides. The monosaccharide may be a conventionally known monosaccharide, such as glucose, galactose, mannose, N-acetylglucosamine, N-acetylgalactosamine and N-acetylmannosamine. The oligosaccharides may include those of the above monosaccharides, chondroitin sulfate, dermatan sulfate and heparin. The polysaccharides may include those obtained by highly polymerizing the above monosaccharides or oligosaccharides. These saccharides may contain sulfate groups or phosphate groups. When [Z] and [D] are saccharide residues, it is preferred that [Z] and [D] be different saccharide residues.

The substituted hydroxyl group is intended to refer to a hydroxyl group whose hydrogen is substituted by a substituent. Specific examples of the substituted hydroxyl group are as described previously.

The bonding style between the saccharide residue represented by [D] and 6-sulfated-N-acetylglucosamine

residue ($6SO_3$-GlcNAc) is such that the position 1 of ($6SO_3$-GlcNAc) is linked to the 2 position, 3 position, 4 position or 6 position of [D]. One of the hydroxyl groups at 2 position, 3 position, 4 position and 6 position of [D] to which ($6SO_3$-GlcNAc) is not linked may be sulfated or phosphated. When [D] is N-acetylglucosamine residue or the like residue, ($6SO_3$-GlcNAc) is linked to the position other than the 2 position, namely to the 3 position, 4 position or 6 position of [D], because the 2-position is blocked.

The sulfated saccharide represented by the above general formula (IIa) serves as a saccharide chain acceptor in the above reaction. By reacting the sulfated saccharide with HO-[GlcNAc]-[Z] as a saccharide chain donor, the sulfated saccharide chain can be introduced to the reducing terminal thereof for extending the chain. As a preferred sulfated saccharide used as the saccharide chain acceptor in the present invention, there may be mentioned p-nitrophenylβ-D-(6-sulfo)-N-acetylglucosamine.

In the formula HO-[GlcNAc]-[Z], [Z] is linked to the 1 position of (GlcNAc). When [Z] is a saccharide residue, the linkage between [Z] and (GlcNAc) may be either α-linkage or β-linkage, in which (GlcNAc) is linked at its 1 position to the 2 position, 3 position, 4 position or 6 position of [Z]. One of the hydroxyl groups at 2 position, 3 position, 4 position and 6 position of [Z] to which ($6SO_3$-GlcNAc) is not linked may be sulfated or phosphated. When [Z] is N-acetylglucosamine residue or the like residue in which the 2-position is blocked, () is linked to the position other than the 2 position, namely to the 3 position, 4 position or 6 position of [Z].

As a preferred saccharide chain donor used in the present invention, there may be mentioned p-nitrophenyl-β-acetylglucosamine ((GlcNAc)-[p-nitrophenyl]).

In the present invention, the sulfated saccharide represented by the formula HO-($6SO_3$-GlcNAc)-[D] is reacted with the saccharide represented by the general formula HO-[GlcNAc]-[Z] to transfer the saccharide chain HO-(GlcNAc) of the HO-[GlcNAc]-[Z] to the sulfated saccharide chain ($6SO_3$-GlcNAc) of the HO-($6SO_3$-GlcNAc)-[D], thereby obtaining the sulfated saccharide of the above general formula (II).

The amount of the raw materials is generally 1:1 in molar ratio, though one of them can be used in an excess amount. The reaction is performed in a liquid phase at a temperature in the range of 0 to 80°C, preferably between room temperature and 50°C. N-Acetylhexosaminidase (E.C. 3.2.1.52) or N-acetylglucosaminidase (E.C. 3.2.1.52) is used as an enzyme. The enzyme may be derived from mold, bovine internal organs, bacteria, bean, yeast, recombinant DNA, etc. The enzyme may be used in a state in which it is supported on a support. When the enzyme is supported on a support, the process can be carried out at low costs. As the support, there may be mentioned an alginic acid resin and liposome.

The reaction is carried out in the presence of a solvent which is generally water. After completion of the reaction, the reaction product is isolated with a yield of 1 to 80 % based on the raw materials. After completion of the reaction, the reaction product is separated and purified by means of reverse chromatography, molecular sieves (gel filtration) or ion exchange.

In the above reaction, a customarily employed buffer solution such as phosphate butter, TRIS buffer or HEPES buffer. In the buffer solution, a hydrophilic solvent such as acetonitrile or methanol may be added in an amount of 0 to 90 %, preferably 10 to 50 %. Although a rare case, a solvent such as toluene or diethyl ether is used. In this case, the reaction is in a two-phase system and is performed at a temperature in the range of 0 to 80°C, preferably between about room temperature and 50°C.

The second sulfated saccharide chain according to the present invention is represented by the following general formula (II) :

$$\text{HO - (GlcNAc) - (6SO}_3\text{ - GlcNAc) - [D]} \qquad \text{(II)}$$

wherein [D] has the same meaning as above.

The bonding style between (GlcNAc) and ($6SO_3$-GlcNAc) may be either α-linkage or β-linkage, in which (GlcNAc) is linked at its 1 position to the 3 position or 4 position of ($6SO_3$-GlcNAc). Namely, the sulfated saccharide chain of the present invention is a saccharide compound which is characterized by having the sulfated saccharide chain in the reducing terminal of the saccharide.

The sulfated saccharide chain of the above general formula (II) effectively bonds to a selectin protein which is present in leukocytes and hence has anti-inflammatory activity. The compound is expected to be widely used in the medicinal field, etc.

Specific example of the sulfated saccharide of the present invention is represented by the following formula:

$$\beta\text{-D-GlcNAc-(1}\rightarrow\text{4)-}\beta\text{-D-(6-sulfo)-GlcNAc-OC}_6\text{H}_4\text{NO}_2\text{-p}$$

Example

Embodiments of the present invention will be next described. The present invention, however, is not limited to these embodiments. The substances obtained are confirmed by NMR.

Example 1

Synthesis of compound of general formula (I):

In a 50 mM sodium phosphate buffer solution (pH 6.0; 1 mL), sodium p-nitrophenylβ-D-(6-sulfo)-N-acetylglucosamine (54 mg) and α-D-glucopyranoside (500 mg) were dissolved, to which *Aspergillus oryzae*-derived β-D-N-acetylhexosaminidase (10.5 units) (EC.3.2.1.52, Sigma Inc.) was added. The mixture was incubated at 35°C for 71 hours. The reaction liquid is then immersed in water at 100°C for 5 minutes to stop the reaction. The reaction mixture was then purified on DEAE Sephadex A-25 column (eluent: water→0.2M ammonium acetate solution). This was further purified on Dowex-50W-X8 column ($H^+$) and finally by Bio-Gel P-2 chromatography, thereby obtaining 9.8 mg of β-D-(6-sulfo)-GlcNAc-(1→4)-α-Glc-$OCH_3$ as a desired product.

$^1$H NMR (400MHZ, $D_2O$) : δ 5.187 (d, 1 H, $J_{1,2}$ 3.2 Hz, H-1), 4.70 4 (d, 1 H, $J_{1,2}$ 8.4 Hz, H-1'), 3.403 (s, 3H,OMe), and2.032(3H, Ac).

$^{13}$C NMR: δ 176.23,100.98,96.63,77.66,75.61,74.79,73.23,72. 92,72.38,71.52,71.25,62.26,56.71,55.79,23.58.

$[\alpha]_D$+27.5° (C=0.47, $H_2O$)

FAB-MS 522$(M+Na)^+$

Example 2

Synthesis of compound of general formula (I):

In a 50 mM sodium phosphate buffer solution (pH 6.0; 1.4 mL), sodium p-nitrophenylβ-D-(6-sulfo)-N-acetylglucosamine (40 mg) and lactose(galactopyranosylβ1→4glucopyranoside (510 mg) were dissolved, to which 1.2 mL of *bovine epididyms* (calf)-derived β-D-N-acetylglucosaminidase (EC.3.2.1.52, Sigma Inc.; 5 units/4.2 mL) was added. The mixture was incubated at 35°C for 29 hours. The reaction liquid is then immersed in water at 100°C for 5 minutes to stop the reaction. The reaction mixture was then purified successively on C-19 ODS column, DEAE Sephadex A-25 column, Dowex-50W-X8 column ($H^+$) and finally Bio-Gel P-2 column, thereby obtaining 16 mg of (6-sulfo)-GlcNAcβ-(1→3)-Galβ1→4Glc as a desired product.

$^1$H NMR ($D_2O$): δ 5.188 (d, 1 H, $J_{1,2}$ 3.6 Hz, H-1α), 4.733 (H-1 "), 4.710 (H-1'), 4.689(H-1β), and 2.032 (3 H, Ac).

Example 3

Synthesis of compound of general formula (I):

In a 50 mM sodium phosphate buffer solution (pH 6.0; 2.0 mL), sodium p-nitrophenylβ-D-(6-sulfo)-N-acetylglucosamine (50 mg) and β-D-N-acetylglucosamine (250 mg) were dissolved, to which 0.5 mL of *Aspergillus oryzae*-derived β-D-N-acetylhexosaminidase (EC.3.2.1.52, Sigma Inc.; 30 units/500 μL) was added. The mixture was incubated at 35°C for 370 hours. The reaction liquid is then immersed in water at 100°C for 5 minutes to stop the reaction. The reaction mixture was then purified successively on C-18 ODS column, DEAE Sephadex A-25 column, Dowex-50W-X8 column ($H^+$) and finally Bio-Gel P-2 column, thereby obtaining 7.3 mg (38 %) of (6-sulfo)-GlcNAc-β(1→4) -GlcNAc as a desired product.

400MHZ-$^1$H NMR ($D_2O$) : δ 5.180 (d, 1 H, $J_{1,2}$ 2.4 Hz, H-1α), 4.6 93 (d, 1 H, $J_{1,2}$ 7.6 Hz, H-1β), 4.591 (d, 1 H, $J_{1',2'}$ 8.4 Hz, H-1'α),), 4.582(d, 1 H, $J_{1',2'}$ 8.4 Hz, H-1'β), and 2.054 and 2.032 (3 H, Ac X 2).

$[\alpha]_D$+12° (C=1.3, $H_2O$)

FAB-MS 548$(M+Na)^+$

Example 4

Synthesis of compound of general formula (II):

In a 50 mM sodium phosphate buffer solution (pH 6.0; 10 mL), p-nitrophenylβ-D-N-acetylglucosamine (100 mg) and p-nitrophenylβ-D-(6-sulfo)-N-acetylglucosamine (800 mg) were dissolved, to which *Aspergillus oryzae*-derived β-D-N-acetylhexosaminidase (3 units, Sigma Inc.) was added. The mixture was incubated at 35°C for 7 hours. The

reaction liquid is then immersed in water at 100°C for 5 minutes to stop the reaction. The reaction mixture was then purified directly on C-18 ODS column. Fractions containing the product and unreacted acceptor were further purified on Toyopearl HW-40S using 25 % methanol solution as an eluent, thereby obtaining 25 mg (14 %) of β-D-GlcNAc-(1→4)-β-D-(6-sulfo)-GlcNAc-$OC_6H_4NO_2$-p as a desired product.

$^1$H NMR ($D_2O$) : δ 8.251 (d, 2 H, J 9.2 Hz, m-Ph), 7.191 (d, 2 H, J 9.2 Hz, o-Ph), 5.325 (d, 1 H, $J_{1,2}$ 8.4, H-1), 4.658 (d, 1 H, $J_{1',2'}$ 8.4, H-1') , 2.102, 2.005 (6H, 2 Ac).

$[\alpha]_D$-28° (C=0.6, $H_2O$)

FAB-MS 670 $(M+Na)^+$

Industrial Applicability

According to the present invention, by using N-acetylhexosaminidase which is a commercially available enzyme for (1) the reaction of a sulfated saccharide as a donor with another saccharide as an acceptor can produce a novel sulfated saccharide in which a sulfated saccharide chain is introduced to a non-reducing terminal for extending the chain and for (2) the reaction of a sulfated saccharide as an acceptor with another saccharide as a donor can produce a novel sulfated saccharide in which a sulfated saccharide chain is introduced to the reducing terminal for extending the chain.

These novel products effectively bond to selectin protein which is present in leukocytes and hence has anti-inflammatory activity. They are expected to be widely used in the medicinal field, etc.

## Claims

**1.** A sulfated saccharide represented by the following general formula (I):

$$[A] - (6SO_3 - GlcNAc) - [C] - R \qquad (I)$$

wherein ($6SO_3$ - GlcNAc) represents a 6-sulfated N-acetylglucosamine residue, [A] represents a hydroxyl group or a saccharide residue, [C] represents a saccharide residue and R represents a hydroxyl group or a substituted hydroxyl group.

**2.** A sulfated saccharide as recited in claim 1, wherein the saccharide residue represented by [A] of the general formula (I) is a saccharide residue derived from a saccharide selected from glucose, galactose, mannose, N-acetylglucosamine, N-acetylgalactosamine, N-acetylmannosamine and oligosaccharides thereof.

**3.** A sulfated saccharide as recited in claim 1 or 2, wherein the substituted hydroxyl group represented by [C] of the general formula (I) is p-nitrophenoxy group.

**4.** A sulfated saccharide as recited in any one of claims 1 through 3, wherein the saccharide residue represented by [A] contains a sulfate group or a phosphate group.

**5.** A sulfated saccharide as recited in any one of claims 1 through 4, wherein the saccharide residue represented by [C] of the general formula (I) is a saccharide residue derived from a saccharide selected from glucose, galactose, mannose, N-acetylglucosamine, N-acetylgalactosamine, N-acetylmannosamine and oligosaccharides thereof.

**6.** A sulfated saccharide as recited in claim 5, wherein the saccharide residue represented by [C] contains a sulfate group or a phosphate group.

**7.** A sulfated saccharide represented by the following general formula (II):

$$HO - (GlcNAc) - (6SO_3 - GlcNAc) - [D] \qquad (II)$$

wherein ($6SO_3$ - GlcNAc) represents a 6-sulfated N-acetylglucosamine residue, (GlcNAc) represents an N-acetylglucosamine residue, and [D] represents a hydroxyl group, a substituted hydroxyl group or a saccharide residue.

**8.** A sulfated saccharide as recited in claim 7, wherein the saccharide residue represented by [D] of the general formula (II) is a saccharide residue derived from a saccharide selected from glucose, galactose, mannose, N-acetylglucosamine, N-acetylgalactosamine, N-acetylmannosamine and oligosaccharides thereof.

**9.** A sulfated saccharide as recited in claim 7 or 8, wherein the substituted hydroxyl group represented by [D] of the general formula (II) is p-nitrophenoxy group.

**10.** A sulfated saccharide as recited in any one of claims 7 through 9, wherein the saccharide residue represented by [D] contains a sulfate group or a phosphate group.

**11.** A process of preparing a sulfated saccharide represented by the following general formula (I):

$$[A] - (6SO_3 - GlcNAc) - [C] - R \qquad (I)$$

wherein ($6SO_3$ - GlcNAc) represents a 6-sulfated N-acetylglucosamine residue, [A] represents a hydroxyl group or a saccharide residue, [C] represents a saccharide residue and R represents a hydroxyl group or a substituted hydroxyl group, **characterized in that** a sulfated saccharide represented by the following general formula (Ia):

$$[A] - (6SO_3 - GlcNAc) - [B] \qquad (Ia)$$

wherein ($6SO_3$ - GlcNAc) and [A] have the same meaning as above and [B] represents a hydroxyl group or a substituted hydroxyl group,
is reacted with a saccharide of the following general formula (Ib):

$$HO - [C] - R \qquad (Ib)$$

wherein [C] represents a saccharide residue and R represents a hydroxyl group or a substituted hydroxyl group, in the presence of N-acetylhexosaminidase or N-acetylglucosaminidase.

**12.** A process of preparing a sulfated saccharide represented by the following general formula (II):

$$HO - (GlcNAc) - (6SO_3 - GlcNAc) - [D] \qquad (II)$$

wherein ($6SO_3$ - GlcNAc) represents a 6-sulfated N-acetylglucosamine residue, (GlcNAc) represents an N-acetylglucosamine residue, and [D] represents a hydroxyl group, a substituted hydroxyl group or a saccharide residue, **characterized in that** a sulfated saccharide represented by the following general formula (IIa):

$$HO - (6SO_3 - GlcNAc) - [D] \qquad (IIa)$$

wherein ($6SO_3$ - GlcNAc) and [D] have the same meaning as above,
is reacted with a saccharide of the following general formula (IIb):

$$HO - [GlcNAc] - [Z] \qquad (IIb)$$

wherein [GlcNAc] represents an N-acetylglucosamine residue and [Z] represents a hydroxyl group, a substituted hydroxyl group or a saccharide residue,
in the presence of N-acetylhexosaminidase or N-acetylglucosaminidase.

## INTERNATIONAL SEARCH REPORT

International application No.
PCT/JP02/02716

A. CLASSIFICATION OF SUBJECT MATTER
Int.Cl$^7$ C12P19/64, C07H11/00, 15/04, 15/203, C08B37/00

According to International Patent Classification (IPC) or to both national classification and IPC

B. FIELDS SEARCHED

Minimum documentation searched (classification system followed by classification symbols)
Int.Cl$^7$ C12P19/00-19/64, C07H11/00, 15/04, 15/203, C08B37/00

Documentation searched other than minimum documentation to the extent that such documents are included in the fields searched

Electronic data base consulted during the international search (name of data base and, where practicable, search terms used)
REGISTRY/CA(STN), BIOSIS/MEDLINE/WPIDS(STN)

C. DOCUMENTS CONSIDERED TO BE RELEVANT

| Category* | Citation of document, with indication, where appropriate, of the relevant passages | Relevant to claim No. |
|---|---|---|
| P,X<br>P,A | ZENG X et al., Synthesis of sulfated disaccharides by use of the transglycosylation of β-D-N-acetylhexosaminidase. The Japanese Society of Carbohydrate Research Nenkai Yoshishu, 02 July, 2001 (02.07.01), page 147 | 1-6,11<br>7-10,12 |
| X<br>A | EP 795560 A1 (Seikagaku Corp.), 17 September, 1997 (17.09.97), & JP 8-518573 A & WO 96/16973 A1 | 1-6<br>7-12 |
| X<br>A | EP 798385 A2 (Seikagaku Kogyo Co., Ltd.), 01 October, 1997 (01.10.97), & JP 9-263595 A & US 5955325 A | 1-6<br>7-12 |
| X<br>A | EP 943688 A2 (Seikagaku Corp.), 22 September, 1999 (22.09.99), & JP 11-313684 A & US 6037159 A | 1-6<br>7-12 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 July, 2002 (03.07.02) | 16 July, 2002 (16.07.02) |
| Name and mailing address of the ISA/<br>Japanese Patent Office | Authorized officer |
| Facsimile No. | Telephone No. |

Form PCT/ISA/210 (second sheet) (July 1998)

| INTERNATIONAL SEARCH REPORT | International application No. PCT/JP02/02716 |
|---|---|

C (Continuation). DOCUMENTS CONSIDERED TO BE RELEVANT

| Category* | Citation of document, with indication, where appropriate, of the relevant passages | Relevant to claim No. |
|---|---|---|
| X<br>A | CHUN-HUNG L et al., Enzymatic synthesis and regeneration of 3'-phosphoadenosine 5'-phosphosulfate (PAPS) for regioselective sulfation of oligosaccharides. J.Am.Chem.Soc., 1995, 117, pages 8031 to 8032 | 7-10<br>1-6,11,12 |

Form PCT/ISA/210 (continuation of second sheet) (July 1998)